# EUROPEAN PATENT APPLICATION

(11) **EP 1 153 620 A2**
(43) Date of publication of application: **14.11.2001**
(21) Application number: 01304250.2
(22) Date of filing: 11.05.2001
(51) Int. Cl.: A61L 15/28, A61K 33/26

(54) **Solid compositions exhibiting selective binding to dissolved iron**

(30) Priority: 12.05.2000 US 569555
(71) Applicant: Johnson & Johnson Medical Ltd., Edinburgh EH2 4NH (GB)
(72) Inventor: Schmidt, Richard, Barnoldswick, Lancashire, BB18 6HJ (GB); Bogan, Declan, Santry, Dublin (IE); Moore, Johnathan, Bradford, West Yorkshire BD3 0HE (GB)
(74) Representative: James, Anthony Christopher W.P.

(57) **Abstract**

The invention provides solid, porous, substantially insoluble composition comprising at least 25% by weight of an oxidized cellulose and having iron bonded thereto. The invention also provides a method of sequestering dissolved iron from an aqueous solution by contacting the solution with a porous, substantially insoluble solid containing at least 25% by weight of an oxidised cellulose. The selective iron-binding property of oxidized cellulose is used in the treatment of bacterial infections.

## Description

### Field

The present invention relates to compositions containing oxidized cellulose selectively bound to iron, processes suitable for the preparation of such compositions, and the use of such compositions in therapeutic applications.

### Background

In the complex biochemistry of infection, there is also thought to be an important role for iron (Fe²⁺/Fe³⁺). Bacteria require iron for metabolism, and can even secrete siderophores for the purpose of scavenging iron. It therefore appears that removal of free iron from infected tissue, preferably without removal of other dissolved species such as Zn²⁺ that are required for metabolism of the host organism, could assist in the treatment of bacterial infection.

It is an object of the present invention to provide an improved biologically acceptable solid material that binds iron strongly from aqueous solution.

It is a further object of the present invention to provide such an iron-binding solid material that has relatively low affinity for Zn²⁺.

It is a further object of the present invention to provide uses of such an iron-binding solid material in methods of medical treatment of infection.

The present invention provides a solid, porous, substantially insoluble composition comprising at least 25% by weight of an oxidized cellulose and having ionic iron bonded thereto.

Preferably, solid composition comprises from 1 to 10,000 ppm by weight of iron bonded thereto, and more preferably from 10 to 1000 ppm by weight of iron bonded thereto.

Typically, the iron is thought to comprise Fe²⁺ or Fe³⁺ complexed to carboxylate groups of the oxidized cellulose.
The present invention also provides a method of sequestering dissolved iron from an aqueous solution by contacting the solution with a porous, substantially insoluble solid containing at least 25% by weight of an oxidised cellulose.

The present invention also provides the use of a porous, substantially insoluble solid containing at least 25% by weight of an oxidised cellulose for the preparation of a medicament for the treatment of a medical condition mediated by dissolved iron. Typically, the medical condition is a bacterial infection.

In a further aspect the present invention provides a method of treatment of a bacterial infection in a mammal comprising administering to the infected tissue a therapeutically effective amount of a porous, substantially insoluble solid containing at least 25% by weight of an oxidised cellulose.

It has been found that the carboxylate groups on the oxidized cellulose provide an effective and selective ligand for the removal of iron from solution. Still more surprisingly, the oxidized cellulose selectively binds to iron over zinc.

The term "oxidized cellulose" refers to any material produced by the oxidation of cellulose, for example with dinitrogen tetroxide. Such oxidation converts primary alcohol groups on the saccharide residues to carboxylic acid groups, forming uronic acid residues within the cellulose chain. The oxidation generally does not proceed with complete selectivity, and as a result hydroxyl groups on carbons 2 and 3 are occasionally converted to the keto form. These keto units introduce an alkali label link, which at pH 7 or higher initiates the decomposition of the polymer via formation of a lactone and sugar ring cleavage. As a result, oxidized cellulose is biodegradable and bioabsorbable under phsyiological conditions.

The preferred oxidized cellulose for practical applications is oxidized regenerated cellulose (ORC) prepared by oxidation of a regenerated cellulose, such as rayon. It has been known for some time that ORC has haemostatic properties. ORC has been available as a haemostatic product called SURGICEL (Registered Trade Mark of Johnson & Johnson Medical, Inc.) since 1950. This product is produced by the oxidation of a knitted rayon material.

A modification of porosity, density and knit pattern led to the launch of a second ORC fabric product, INTERCEED (Registered Trade Mark of Johnson & Johnson Medical, Inc.), which was shown to reduce the extent of post-surgical adhesions in abdominal surgery.

WO98/00180 describes the use of ORC and complexes thereof for the treatment of chronic wounds, such as diabetic ulcers. The mechanism of action of the ORC in chronic wound treatment is thought to involve binding and inactivation of matrix metalloproteinase enzymes present in the wound fluid.

WO98/00446 describes the preparation of ORC oligosaccharides by partial hydrolysis of ORC in alkali solution, followed by dialysis and purification. The ORC oligosaccharides are shown to have similar matrix metalloproteinase binding properties to ORC, and are also indicated for the treatment of chronic wounds.

In the use according to the present invention, the oxidized cellulose preferably comprises oxidized regenerated cellulose. The ORC may be in the form of fibers or woven or nonwoven fabrics or freeze-dried or solvent-dried sponges. Preferably, at least 40% by weight of the solid material consists of oxidized regenerated cellulose

In preferred embodiments of the present invention, the oxidized cellulose is complexed with collagen to form structures of the kind described in WO98/00180 and WO98/00446, the entire contents of which are expressly incorporated herein by reference. For example, the oxidized cellulose may be in the form of milled ORC fibres that are dispersed in a freeze-dried collagen sponge. This provides for certain therapeutic and synergistic effects arising from the complexation with collagen.

Preferably, the solid composition containing oxidized polysaccharide according to the present invention is substantially insoluble in water. That is to say, it has a solubility of less than 1g/l in water at 25 °C. Low solubility renders such polysaccharides especially suitable for use to remove iron from biological fluids.

The solid composition used in the present invention preferably selectively complexes with Fe³⁺ over Zn²⁺. More preferably, the stability constant of the polysaccharide complex with Fe³⁺ ions is at least five times the stability constant of the polysaccharide complex Zn²⁺ ions. Preferably, a complex of the polysaccharide with Fe³⁺ in water has a stability constant of at least 10⁶, preferably at least 10⁹.

### Brief Description of the Drawings

Particular embodiments of the present invention will now be described further, by way of example, with reference to the accompanying drawings, in which:-
Figure 1 shows an elution plot of dissolved Fe and Zn produced in accordance with Procedure 1 and showing eluted Fe and Zn for the control serum, the control serum with added Fe and Zn at 50 ppm each and the same serum after contacting with an ORC cloth (SURGICEL);
Figure 2 shows an elution plot of dissolved Fe and Zn produced in accordance with Procedure 1 and showing eluted Fe and Zn for the control serum, the control serum with added Fe and Zn at 50 ppm each and the same serum after contacting with a collagen/ORC sponge ;
Figure 3 shows an elution plot of dissolved Fe and Zn produced in accordance with Procedure 1 and showing eluted Fe and Zn for the control serum, the control serum with added Fe and Zn at 50 ppm each and the same serum after contacting with a collagen/alginate FIBRACOL sponge; and
Figure 4 shows an elution plot of dissolved Fe and Zn produced in accordance with Procedure 1 and showing eluted Fe and Zn for the control serum, the control serum with added Fe and Zn at 50 ppm each and the same serum after contacting with a collagen sponge.

### Detailed Description

### Example 1

A sample of a commercially available knitted ORC cloth (registered trade mark SURGICEL of Johnson & Johnson Medical, Arlington) was provided.

### Example 2

A collagen/ORC sponge is prepared in similar fashion to the methods described in WO98/00180. Briefly, purified collagen is suspended in 0.05m acetic acid. Milled ORC powder (milled SURGICEL cloth) is added to the suspension at a weight ratio of 45:55 ORC:collagen to give a total solids concentration of about 0.67% by weight, and the mixture is homogenized. The complex suspension is degassed in a vacuum oven for 10 minutes, and is then poured into a tray and frozen to -40°C. The frozen suspension is then freeze-dried and dehydrothermally cross-linked using a programmable freeze-drier with a temperature ramping facility.

### Example 3 (comparative)

A sample of a commercially available collagen/alginate sponge produced by freeze drying a slurry of collagen and alginate substantially as described in US patent 4,614,794 was obtained. The product is commercially available under the registered trade mark FIBRACOL from Johnson & Johnson Medical, Arlington.

### Example 4 (comparative)

A freeze dried collagen sponge was prepared substantially as described in Example 1, but omitting the ORC from the slurry.

### Procedure 1

The ability of the materials to sequester iron ions selectively over zinc ions in aqueous media according to the present invention is determined as follows.

The solid material is added with stirring to a solution containing iron and/or zinc ions in a suitable matrix, such as buffer, serum-containing buffer or cell culture medium. Following a suitable incubation period, ion exchange chromatography is used to determine the levels of uncomplexed Fe³⁺ and/or Zn²⁺ ions remaining in solution.

In the present research, solutions of FeCl₃.6H2O and ZnSO₄.7H₂O, each at 50 ppm, are dissolved in tris-HCL at pH 7.4 with 10% calf serum. The mixed solution (10ml) is incubated with 100 mg of the solid material at 37°C with gentle agitation on a shaker table for 16 hours. The samples were then centrifuged, and a 0.9ml sample of the supernatant was then analysed for ion or zinc content, by the following method.

The centrifuged solution was treated with 0.1ml of a 20% w/v solution of trichloroacetic acid (TCA) to give a final concentration of 2% w/v TCA. The tube was vortexed for 10 seconds and then centrifuged for 15 minutes or longer to remove solids. The supernatant (0.5ml) was added to a clean dry HPLC vial, to which was added 0.5ml of 0.5 molar nitric acid prepared in deionised water.

The samples were then analysed for free iron and zinc in a Dionex DX500 HPLC apparatus using the following method details:
- Instrument:: Dionex DX500 HPLC
- Column:: lonpac CS5A Analytical Column (Dionex part no. 046100)
lonpac CS5A Guard Column (Dionex part no. 046104)
- Eluents:: A Deionised water
B Metpac PDCA reagent concentrate (part no. 046088)
C Metpac PAR reagent diluent (Dionex part no. 046094)
- Postcolumn Reagent:: 4-(2-pyridylazo) resorcinol monosodium salt (PAR) 0.12g/l
- Detector Wavelength:: 530 nm; Temperature: 25 °C
- Flow rate:: 1.2 ml.min (80% eluent A : 20% eluent B) in column; 0.6 ml/min eluent C postcolumn
- Sample:: Approx 100 µl

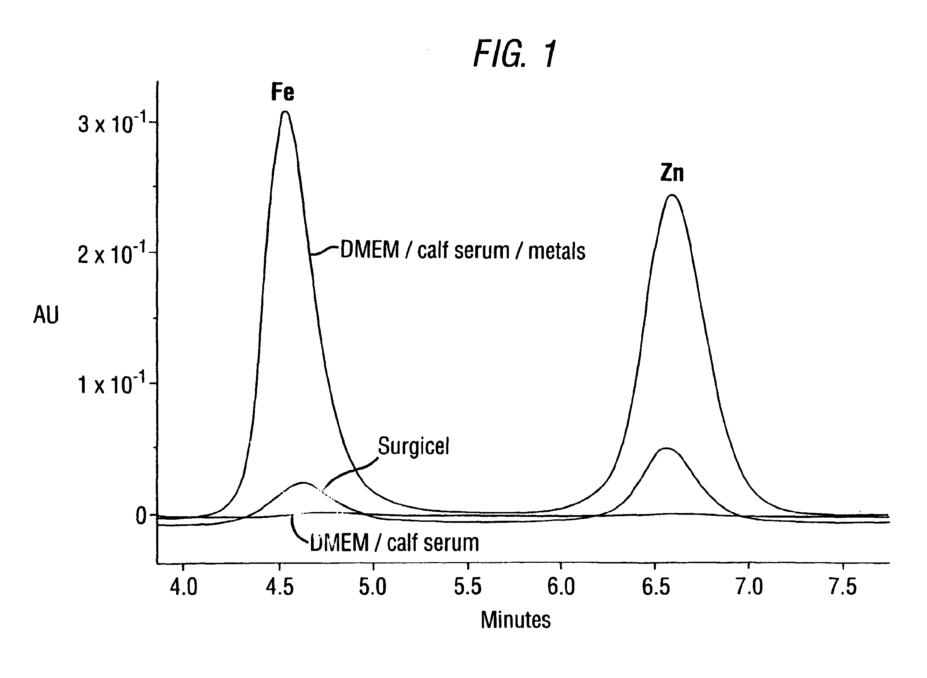

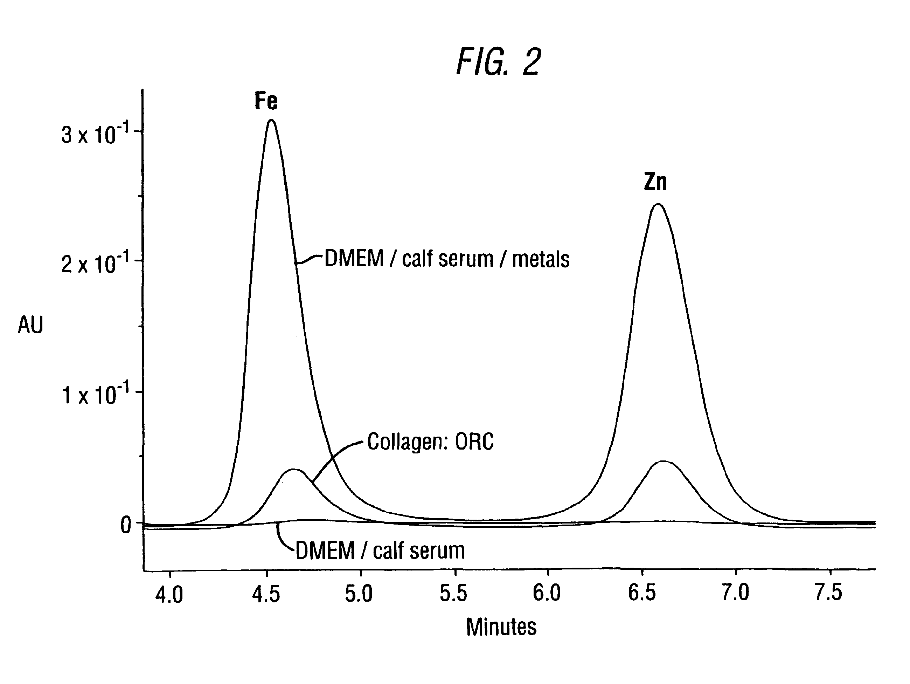

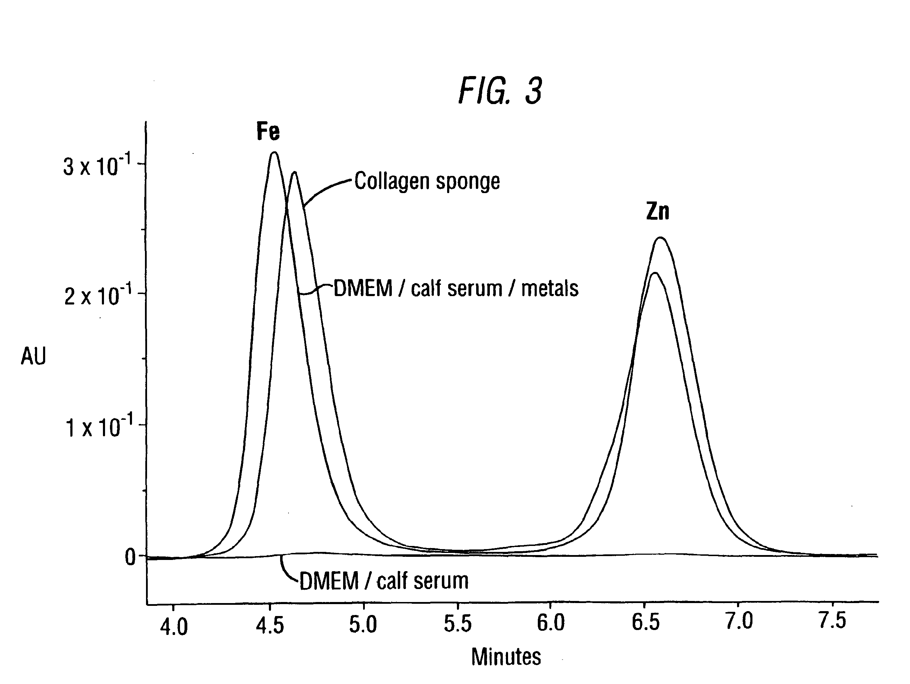

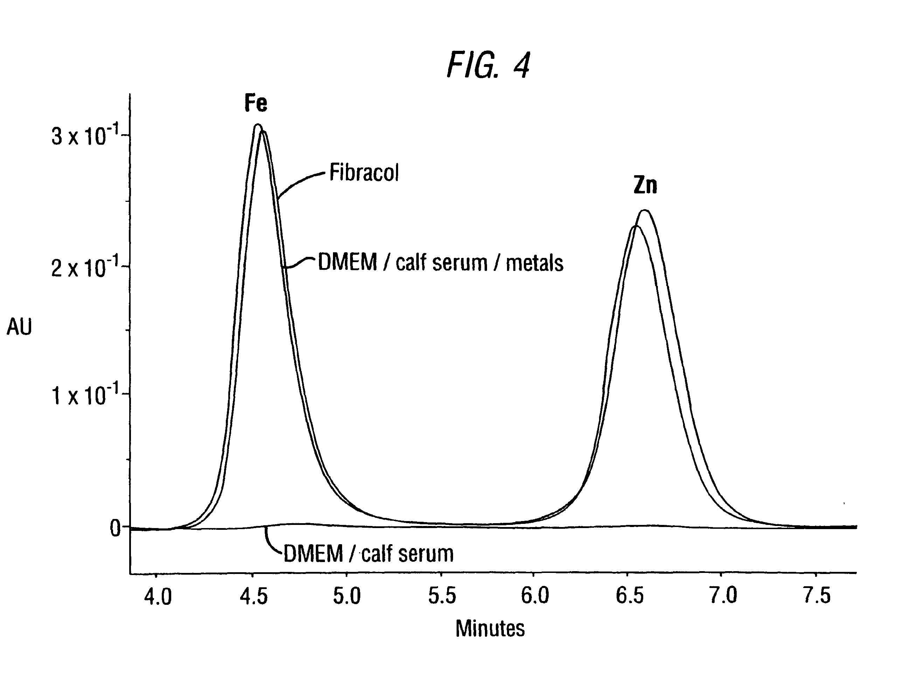

The data for the samples are shown in Figures 1 to 4. It can be seen that the ORC cloth and the collagen/ORC sponcge remove removes Fe³⁺ with selectivity over Zn²⁺. The collagen sponge and the collagen/alginate sponge show very little apparent binding with either Fe³⁺ or Zn²⁺.

The above embodiments have been described by way of example only. Many other embodiments falling within the scope of the accompanying claims will be apparent to the skilled reader.

## Claims

1. A solid, porous, substantially insoluble composition comprising at least 25% by weight of an oxidized cellulose and having iron bonded thereto.

2. A solid composition according to claim 1, comprising from 1 to 10,000 ppm by weight of iron bonded thereto.

3. A solid composition according to claim 1, wherein the iron comprises Fe²⁺ or Fe³⁺ complexed to carboxylate groups of the oxidized cellulose.

4. A solid composition according to claim 1, wherein the oxidized celulose consists essentially of oxidized regenerated cellulose (ORC).

5. A solid composition according to claim 1, wherein the solid material comprises a freeze-dried sponge of collagen and oxidized regenerated cellulose (ORC).

6. A solid composition according to claim 1, wherein the solid material consists essentially of a freeze-dried sponge of collagen and oxidized regenerated cellulose (ORC).

7. A method of sequestering dissolved iron from an aqueous solution by contacting the solution with a porous, substantially insoluble solid containing at least 25% by weight of an oxidised cellulose.

8. A method according to claim 7, wherein the aqueous solution comprises a biological fluid.

9. A method according to claim 7, wherein the aqueous solution further comprises dissolved zinc, and the porous solid sequesters the dissolved iron preferentially to the dissolved zinc.

10. A solid composition according to claim 7, wherein the oxidized celulose consists essentially of oxidized regenerated cellulose (ORC).

11. A solid composition according to claim 7, wherein the solid material comprises a freeze-dried sponge of collagen and oxidized regenerated cellulose (ORC).

12. A solid composition according to claim 7, wherein the solid material consists essentially of a freeze-dried sponge of collagen and oxidized regenerated cellulose (ORC).

13. Use of a porous, substantially insoluble solid containing at least 25% by weight of an oxidised cellulose for the preparation of a medicament for the treatment of a medical condition mediated by dissolved iron.

14. Use according to claim 13, wherein the medical condition comprises a bacterial infection.

15. A solid composition according to claim 13, wherein the oxidized celulose consists essentially of oxidized regenerated cellulose (ORC).

16. A solid composition according to claim 13, wherein the solid material comprises a freeze-dried sponge of collagen and oxidized regenerated cellulose (ORC).

17. A solid composition according to claim 13, wherein the solid material consists essentially of a freeze-dried sponge of collagen and oxidized regenerated cellulose (ORC).

18. A method of treatment of a bacterial infection in a mammal comprising administering to the infected tissue a therapeutically effective amount of a porous, substantially insoluble solid containing at least 25% by weight of an oxidised cellulose.

19. A solid composition according to claim 18, wherein the oxidized celulose consists essentially of oxidized regenerated cellulose (ORC).

20. A solid composition according to claim 18, wherein the solid material comprises a freeze-dried sponge of collagen and oxidized regenerated cellulose (ORC).

21. A solid composition according to claim 18, wherein the solid material consists essentially of a freeze-dried sponge of collagen and oxidized regenerated cellulose (ORC).
